# EUROPÄISCHE PATENTSCHRIFT

(11) **EP 1 988 587 B1**
(45) Veröffentlichungstag und Bekanntmachung des Hinweises auf die Patenterteilung: **07.12.2016**
(21) Anmeldenummer: 07008747.3
(22) Anmeldetag: 30.04.2007
(51) Int. Cl.: H01L 51/54, C07C 255/51

(54) **Oxokohlenstoff-, Pseudooxokohlenstoff- und Radialenverbindungen sowie deren Verwendung**
Oxocarbon, pseudo oxocarbon and radialene compounds and their use
Composés de matière d'oxyde de carbone, de pseudo oxyde de carbone et de radialène ainsi que leur utilisation

(43) Veröffentlichungstag der Anmeldung: 05.11.2008
(60) Teilanmeldung: 16169129.0
(73) Patentinhaber: Novaled GmbH, 01307 Dresden (DE)
(72) Erfinder: Hartmann, Prof Dr., Horst, 01326 Dresden (DE); Zeika, Olaf, 01099 Dresden (DE); Lux, Andrea, 01277 Dresden (DE); Willmann, Steffen, 01277 Dresden (DE)
(74) Vertreter: Bittner, Thomas L.

(56) Entgegenhaltungen:
- EP-A- 1 596 445
- JP-A- 61 254 582
- US-A- 3 963 769
- US-A- 4 005 091
- US-A- 4 133 821
- US-B2- 8 057 712
- KAZUKO TAKAHASHI ET AL: "NOVEL METALLIC CHARGE-TRANSFER COMPLEXES COMPOSED OF A Ú3RADIALENETYPE ACCEPTOR: A 1,2-BIS(P-BENZOQUINO)-3-Ú2-(DICYANOMETHYLE NE- 2,5-SELENOQUINOCYCLOPROPANE DERIVATIVE" ADVANCED MATERIALS, WILEY VCH, WEINHEIM, DE, Bd. 7, Nr. 7, 1. Juli 1995 (1995-07-01), Seiten 639-641, XP000520477 ISSN: 0935-9648
- TAKEHIRO CHONAN AND KAZUKO TAKAHASHI: "The Synthesis of Difluoro and Dimethyl Derivatives of 2,6- Bis(dicyanomethylene)-2,6-dihydro-4H-cyclo penta(2,1-b:3,4-b0)- dithiophen-4-one (CPDT-TCNQ) and the Conducting Properties of the Metallic Salts Based on the Dimethyl Derivative" BULLETIN OF THE CHEMICAL SOCIETY OF JAPAN, CHEMICAL SOCIETY OF JAPAN, TOKYO, JP, Bd. 77, Nr. 8, 6. August 2004 (2004-08-06), Seiten 1487-1497, XP007902786 ISSN: 0009-2673
- IYODA M ET AL: "Novel synthesis of hexaaryl[3]radialenes via dibromo[3]dendralenes" TETRAHEDRON LETTERS, ELSEVIER, AMSTERDAM, NL, Bd. 41, Nr. 36, September 2000 (2000-09), Seiten 7059-7064, XP004208260 ISSN: 0040-4039
- Brückner, Reinhard: "Reaktionsmechanismen", 2003, Spektrum Akademischer Verlag Heidelberg . Berlin ISBN: 3-8274-1189-0 page 519,

## Beschreibung

Die vorliegende Erfindung betrifft Oxokohlenstoff-, Pseudooxokohlenstoff- und Radialenverbindungen sowie deren Verwendung als organischer Dotand zur Dotierung eines organischen halbleitenden Matrixmaterials zur Veränderung der elektrischen Eigenschaften desselben, als Blockermaterial sowie als Ladungsinjektionsschicht und als Elektrodenmaterial. Ebenfalls betrifft die Erfindung organische halbleitende Materialien sowie elektronische Bauelemente, in denen die Oxokohlenstoff-, Pseudooxokohlenstoff- und Radialenverbindungen eingesetzt werden.

In der vorliegenden Anmeldung werden als Radialene Alicyclen bezeichnet, in denen alle Ringatome sp2-hybridisiert sind und soweit möglich exocyclische C-C-Doppelbindungen tragen, siehe auch H. Hopf und G. Maas, Angew. Chem. (1992), 8, 955. Oxokohlenstoff- und Pseudooxokohlenstoffverbindungen sind als nicht-benzoide Aromaten hinlänglich bekannt, siehe beispielsweise G. Seitz, Nachr. Chem. Tech. Lab. 28 (1980), Seiten 804-807. Die erste Oxokohlenstoff-Verbindung, das krokonsaure Kali, wurde von L. Gmelin 1825 aus Pottasche und Kohle hergestellt. Als Pseudooxokohlenstoffe werden, wie einem Fachmann auf dem Gebiet ohne weiteres bekannt ist, solche Verbindungen bezeichnet, bei denen zumindest ein Sauerstoffatom durch ein anderes Heteroatom ersetzt ist.

Seit einigen Jahren ist bekannt geworden, dass man organische Halbleiter durch Dotierung hinsichtlich ihrer elektrischen Leitfähigkeit stark beeinflussen kann. Solche organischen halbleitenden Matrixmaterialien können entweder aus Verbindungen mit guten Elektronendonator-Eigenschaften oder aus Verbindungen mit guten Elektronenakzeptor-Eigenschaften aufgebaut werden. Zum Dotieren von Elektronendonator-Materialien (HT) sind starke Elektronen-Akzeptoren wie Tetracyanochinondimethan (TCNQ) oder 2,3,5,6-Tetrafluoro-tetracyano-1,4-benzochinondimethan (F4TCNQ) bekannt geworden, M. Pfeiffer, A. Beyer, T. Fritz, K. Leo, Appl. Phys. Lett., 73 (22), 3202-3204 (1998). und J. Blochwitz, M. Pfeiffer, T. Fritz, K. Leo, Appl. Phys. Lett., 73 (6), 729-731 (1998). Diese erzeugen durch Elektronentransferprozesse in elektronendonatorartigen Basismaterialien (Löchertransportmaterialien) sog. Löcher, durch deren Anzahl und Beweglichkeit sich die Leitfähigkeit des Basismaterials mehr oder weniger signifikant verändert. Als Matrixmaterialien mit Löchertransporteigenschaften sind beispielsweise N,N'-perarylierte Benzidine **TPD** oder N,N',N"-perarylierte Starburstverbindungen, wie die Substanz **TDATA,** oder aber auch bestimmte Metallphthalocyanine, wie insbesondere Zinkphthalocyanin **ZnPc** bekannt.

EP 1 596 445 A1 betrifft organische mesomere Verbindungen als Dotanden zur Dotierung von organischen halbleitenden Matrixmaterialien zur Veränderung der elektrischen Eigenschaften derselben. Die Dotierung von organischen Halbleitermaterialien wird hier mit Chinonderivaten erreicht.

Kazuko Takahashi et al., Advanced Materials, 7 (1995) No. 7, Seiten 639-641 beschreibt neue metallische Ladungstransferkomplexe, die einen [3]Radialen-artigen Akzeptor umfassen.

JP 61254582 A betrifft [4]Radialenverbindungen und deren Verwendung in organischen elektrisch leitfähigen Materialien. Die Elektronendonoreigenschaften der besagten Verbindungen wird hervorgehoben.

Masahiko Iyoda et al., Tetrahedron Letters, 41 (2000) 7059-7064 betrifft die Herstellung von Hexaaryl[3]radialenen via Dibrom[3]-Dendralenen.

US 4,133,821 betrifft Alkylidendiquinoncyclopropane und Diarylcyclopropane und die Herstellung der Ersten durch Oxidation Letzterer. Verschiedene Eigenschaften der erhaltenen Verbindungen werden beschrieben.

US 3,963,769 beschreibt Trimethylencyclopropane und deren Herstellung.

US 4,005,091 betrifft die Herstellung asymmetrischer Trimethylencyclopropane, ausgehend von Derivaten der Malonsäure in Gegenwart starker Basen.

Die bisher beschriebenen Verbindungen haben jedoch für eine technische Anwendung Nachteile in der Produktion dotierter halbleitender organischer Schichten oder von entsprechenden elektronischen Bauteilen mit derartigen dotierten Schichten, da die Fertigungsprozesse in großtechnischen Produktionsanlagen oder solchen im Technikumsmaßstab nicht immer ausreichend präzise gesteuert werden können, was zu hohem Steuerungs- und Regelaufwand innerhalb der Prozesse führt, um eine gewünschte Produktqualität zu erzielen, oder zu unerwünschten Toleranzen der Produkte. Ferner bestehen Nachteile bei der Verwendung bisher bekannter organischer Donatoren bezüglich der elektronischen Bauelenzentstrukiuren, wie Leuchtdioden (OLEDs), Feldeffekttransistor (FET) oder Solarzellen selber, da die genannten Produktionsschwierigkeiten bei der Handhabung der Dotanden zu unerwünschten Ungleichmäßigkeiten in den elektronischen Bauteilen oder unerwünschten Alterungseffekten der elektronischen Bauteile führen können. Gleichzeitig ist jedoch zu beachten, dass die zu verwendenden Dotanden extrem hohe Elektronenaffinitäten (Reduktionspotentiale) und andere für den Anwendungsfall geeignete Eigenschaften aufweisen, da beispielsweise die Dotanden unter gegebenen Bedingungen auch die Leitfähigkeit oder andere elektrische Eigenschaften der organisch halbleitenden Schicht mit bestimmen. Entscheidend für den Dotiereffekt sind die energetischen Lagen des HOMO des Matrixmaterials und des LUMO des Dotanden.

Aus EP 1 596 445 A1 sind Chinonderivate bekannt, die jedoch kein Ringsystem zeigen, bei dem jedes Ringkohlenstoffatom exocyclische Doppelbindungen aufweist.

Aus Advanced Materials, Band 7, Nr. 7, Seiten 639-543, JP 61254582 A und Bulletin of the Chemical Society of Japan, Band 77, Nr. 8, Seiten 1487-1497, sind weitere Radialenverbindungen bekannt, die jedoch nicht unter die in der vorliegenden Anmeldung beanspruchten Verbindungen fallen.

Der vorliegenden Erfindung liegt die Aufgabe zugrunde, die Nachteile des Stands der Technik zu überwinden, insbesondere neue organische mesomere Verbindungen bereitzustellen, die insbesondere als Dotand zur Dotierung organischer Halbleiter eingesetzt werden können, die ferner im Produktionsprozeß leichter handhabbar sind und die zu elektronischen Bauteilen führen, deren organische halbleitende Materialien reproduzierbar hergestellt werden können.

Diese Aufgabe wird durch die unabhängigen Ansprüche der vorliegenden Anmeldung gelöst. Bevorzugte Ausführungsformen ergeben sich aus den Unteransprüchen.

Bei den erfindungsgemäßen Verbindungen ist die Lage des LUMO so niedrig, daß weitere technisch interessante Lochtransportmaterialien jetzt erst effizient dotiert werden können. Durch die außergewöhnlich niedrige Lage des LUMO und das damit verbundene hohe Reduktionspotential der Verbindungen können auch Leistungseffizienzen von Solarzellen wesentlich verbessert werden. Zusätzlich sind diese Verbindungen aufgrund ihrer hohen Polarität außergewöhnlich diffusionsstabil in organischen Schichten. Durch höhere Verdampfungstemperatur bzw. geringere Flüchtigkeit unter gleichen Bedingungen können die Produktionsprozesse besser kontrolliert und damit mit geringerem Aufwand und reproduzierbarer durchgeführt werden, wobei durch die Bereitstellung von Oxokohlenstoffen, Pseudooxokohlenstoffen und Radialenen als Dotanden diese in den jeweiligen Bauteilen bei geringen Diffusionskoeffizienten, die zeitlich gleichbleibende Bauelementstruktur gewährleisten, eine ausreichende elektrische Leitfähigkeit der organischen halbleitenden Matrix bei günstiger Elektronenaffinität der Dotanden ermöglichen. Ferner kann durch die Dotanden die Ladungsträgerinjektion von Kontakten in die dotierte Schicht verbessert werden. Ferner kann das dotierte organische Halbleitermaterial bzw. das resultierende elektronische Bauteil aufgrund der erfindungsgemäß verwendeten Verbindungen eine verbesserte Langzeitstabilität aufweisen. Dies betrifft beispielsweise eine Verringerung der Dotandenkonzentration mit der Zeit. Ferner betrifft dies die Stabilität der dotierten Schicht, die benachbart zu undotierten Schichten eines elektrooptischen Bauteils angeordnet ist, so daß elektrooptische Bauteile mit erhöhter Langzeitstabilität der elektrooptischen Eigenschaften, wie Lichtausbeute bei einer vorgegebenen Länge, Wirksamkeit einer Solarzelle oder dergleichen, resultieren.

Die Bedampfungsrate eines Substrats mit den erfindungsgemäß verwendeten Verbindungen kann beispielsweise unter Verwendung eines Quarzdickenmonitors bestimmt wird, wie er beispielsweise bei der Herstellung von OLEDs üblicherweise eingesetzt wird. Insbesondere kann das Verhältnis der Bedampfungsraten von Matrixmaterialien und Dotanden durch unabhängige Messungen derselben unter Verwendung von zwei getrennten Quarzdickenmonitoren gemessen werden, um das Dotierungsverhältnis einzustellen.

Es versteht sich, dass die erfindungsgemäß verwendeten Verbindungen vorzugsweise derart beschaffen sind, dass sie mehr oder weniger oder praktisch unzersetzt verdampfen. Es können unter Umständen jedoch auch zielgerichtet Precursor als Dotandenquelle eingesetzt werden, die die erfindungsgemäß verwendeten Verbindungen freisetzen, beispielsweise Säureadditionssalze, beispielsweise einer flüchtigen oder nichtflüchtigen anorganischen oder organischen Säure, oder Charge-Transfer-Komplexe derselben, wobei die Säuren bzw. Elektronen-Donatoren vorzugsweise nicht oder nur gering flüchtig sind oder der Charge-Transfer-Komplex selber als Dotand wirkt.

Vorzugsweise ist der Dotand derart ausgewählt, dass er unter sonst gleichen Bedingungen wie insbesondere Dotierungskonzentration (Molverhältnis Dotand:Matrix, Schichtdicke, Stromstärke) bei gegebenem Matrixmaterial (beispielsweise Zinkphtalocyanin oder einem anderen weiter unten genannten Matrixmaterial) eine genau so hohe oder vorzugsweise eine höhere Leitfähigkeit erzeugt als F4TCNQ, beispielsweise eine Leitfähigkeit (S/cm) von größer/gleich dem 1,1-fachen, 1,2-fachen oder größer/gleich dem 1,5-fachen oder zweifachen derjenigen von F4TCNQ als Dotand.

Vorzugsweise ist der erfingdungsgemäß verwendete Dotand derart ausgewählt, dass das mit diesem dotierte halbleitende organische Matrixmaterial nach einer Temperaturänderung von 100°C auf Raumtemperatur (20°C) noch ≥ 20%, vorzugsweise ≥ 30%, besonders bevorzugt ≥ 50% oder 60% der Leitfähigkeit (S/cm) des Wertes bei 100°C aufweist.

Im folgenden werden einige Oxokohlenstoffe, Pseudooxokohlenstoffe bzw. Radialene gezeigt:

### Derivate der [3] Radialene

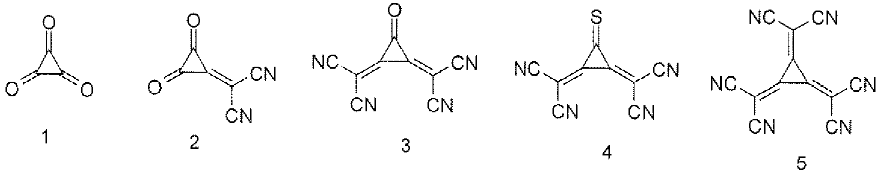

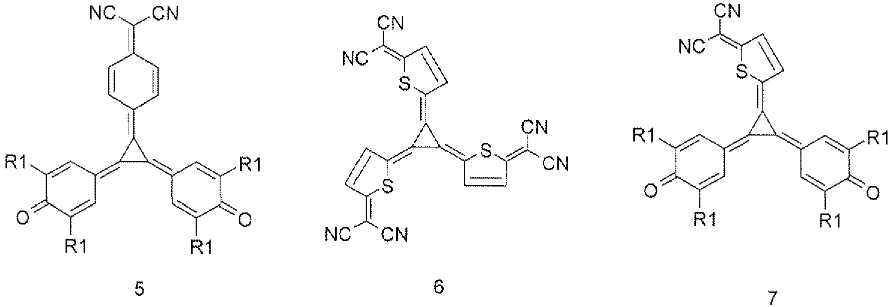

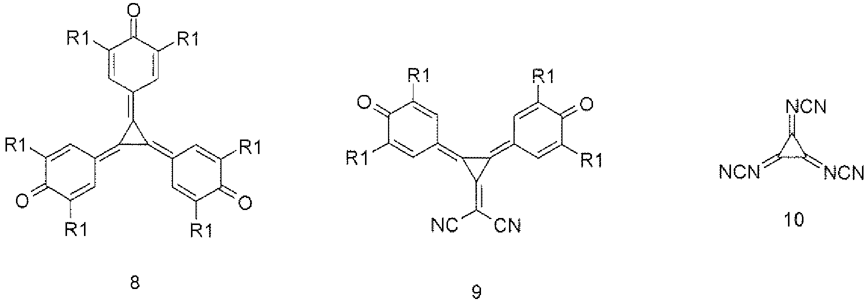

### R1 = Methyl, Iso-Propyl, t-Butyl

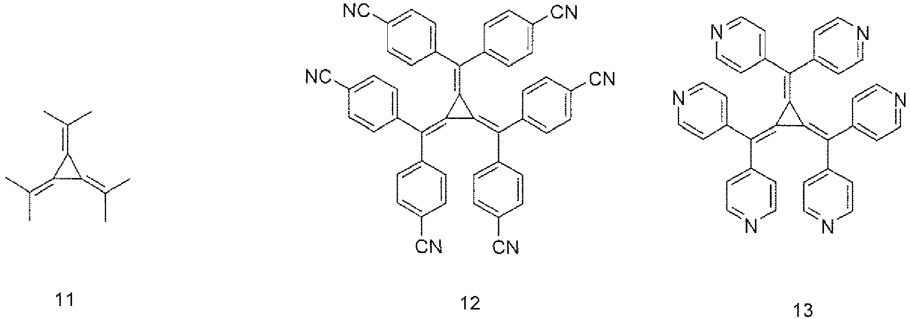

### Derivate der [4] Radialene

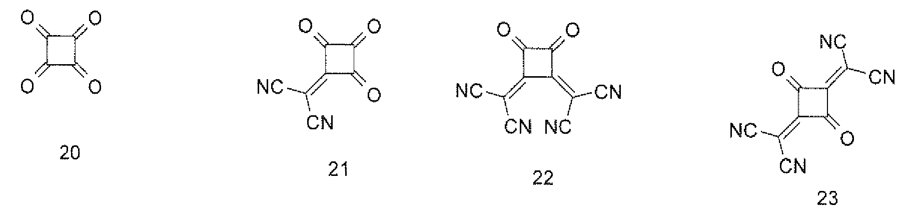

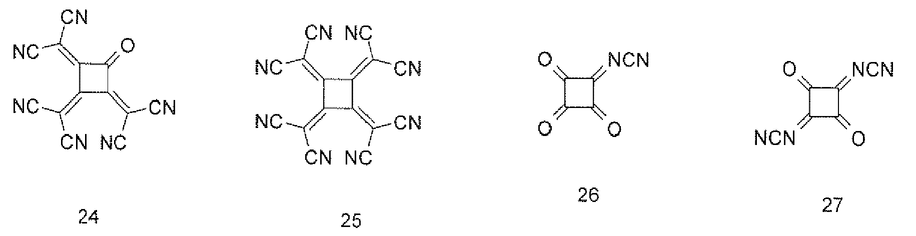

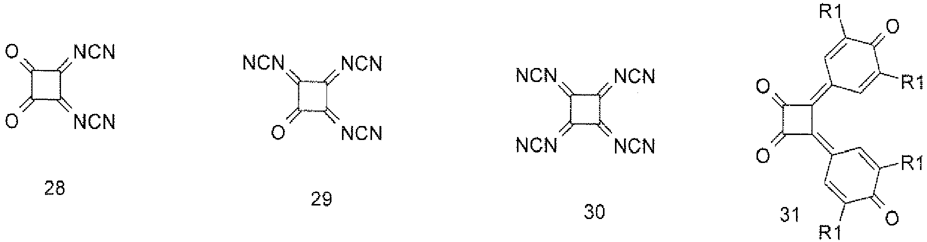

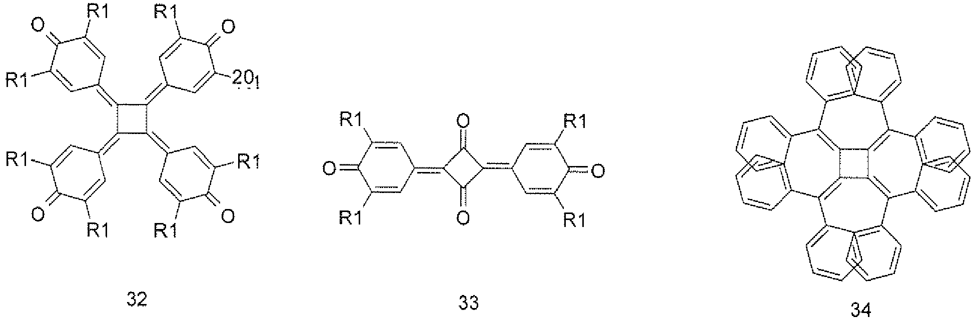

### Derivate der [5] Radialene

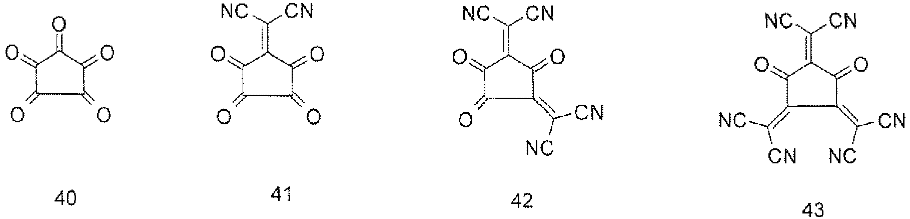

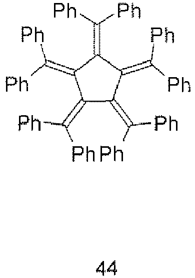

Weitere Derivate der Oxokohlenstoff-, Pseudooxokohlenstoff- bzw. [6] Radialen-Strukturen

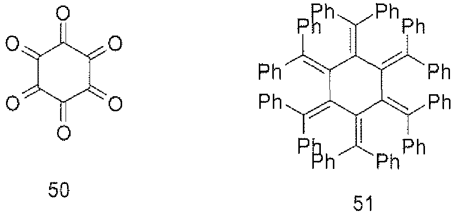

### Darstellung der Oxokohlenstoff-, Pseudooxokohlenstoff- bzw. Radialen-Strukturen

Die erste Oxokohlenstoff-Verbindung das krokonsaure Kali wurde von L.Gmelin 1825 aus Pottasche und Kohle hergestellt. Oxokohlenstoffe und deren Ester und Halogenide reagieren bevorzugt mit elektronenreichen Verbindungen, wie aliphatischen und aromatischen Aminen, Aromaten und Heteroaromaten. A.H.Schmidt, Synthesis (1980) 961. Besonders geeignet sind die Reaktionsprodukte aus Tetrachlorcyclopropen und Phenolen in Gegenwart von Lewissäuren bzw. CH-aciden Verbindungen mittels starker Basen, wie z.B. Arylacetonitrile, 1,3-Diketone, Cyclopentadiene, Malodinitrile, Akzeptor-substituierte Diarylmethane, elektronenarme Diheteroarylmethane. Nach erfolgter Oxidation werden [3]Radialene erhalten, R.West et al. J. Org. Chem. (1975) 40 2295; T.Kazuka, T.Shinji J. Chem. Soc. Chem. Commun.(1994) 519; T.Fukunaga et al JACS (1976) 98 610.

Weiterhin auch sehr gut geeignet sind Quadratsäuredichlorid und Phenole, die anschließend zu 4[Radialenen] oxidiert werden können, R.West, S.K.Koster J.Org.Chem. (1975) 40 2300; das nucleophile Anion des CH-aciden Malonsäuredinitrils lässt sich ebenfalls bevorzugt mit Estern unter Alkoholabspaltung zu dianionischen Quadratsäureverbindungen substituieren, T.Fukanaga J.Am.Chem.Soc. (1976) 98 610; W.Ziegenbein, H.-E.Sprenger Angew. Chem. (1967) 79 581; G.Seitz et al. Chem. Ber. (1987) 120 1691. Die Oxidation dieser CN-substituierten Verbindungen gelang bisher nur elektrochemisch, T.A.Blinka, R.West, Tetrahedron Lett. (1983) 24 1567. Durch thermische Dimerisierung von Dichinonethylenen lassen sich gleichfalls [4]Radialene darstellen, R.West,S.K.Koster, JOC (1975) 40 2300.

Von Fatiadi konnten die ersten Krokonsäurederivate hergestellt werden, die mit Malodinitril substituiert worden sind, J.Org.Chem. (1980) 45 1338, J.Am.Chem.Soc. (1978) 100 2586. Die Oxidation dieser Verbindungen wurde auch von ihm elektrochemisch untersucht, A.J.Fatiadi, L.M.Doane J.Electroanal.Chem. (1982) 135 193-209.

Es sind aber auch [6]Radialene bekannt, H.Hopf, A.K.Wick Helv. Chim. Acta (1961)46 380-6.

Einige spätere Vertreter fanden bzw. finden Anwendung in der Elektrophotographie, als Elektrolumineszenzmaterial in Bildschirmen, als Farbstoff, als Photoleiter, als organische Oxidantien, US 4003943 (1977),JP 07-168377, JP 2004010703 A (2004), US 4133821 (1979).

### Darstellung cyclischer Oxokohlenstoff- bzw. Pseudooxokohlenstoff-Derivate

### a) 1,3-Bis(dicyanomethylen)indan-2-yliden-bis(4-oxo-[3,5-di-t-butyl]-2,5-cyclohexadienyliden)-cyclopropan

4,75 g Bis(4-oxo-[3,5-di-t-butyl]-2,5-cyclohexadienyl)-cyclopropenon, 3,5 g 1,3-Bis(dicyanomethylen)-indan sowie 60mg ß-Alanin werden in 12 ml Acetanhydrid gelöst und kurz am Rückfluß unter Rühren erhitzt. Man versetzt mit 60ml Toluen, lässt abkühlen und saugt den rotbrauen kristallinen Feststoff ab. Anschließend wird mit Benzin/Toluen gewaschen und umkristallisiert.
Ausbeute: 4,6g

Unter Argon werden 2,5g der rotbraunen Kristalle in 100ml Chloroform gelöst und mit einer Lösung aus 4,7g rotem Blutlaugensalz und 8,8g KOH in 150 ml Wasser vereinigt. Nach 1h intensiven Rührens wird die organische Phase mit Na2SO4 getrocknet und eingeengt und das Produkt umkristallisiert.
Ausbeute: 2,3g schwarzgrüne Kristalle Fp.: >250°C unter Zersetzung

### b) Hexa(methoxycarbonyl)trimethylencyclopropan

Zu einer Suspension aus 16,5g 50% NaH in 340ml Glyme werden unter Argon, Eiskühlung und Rühren 24g Dimethylmalonat langsam zugetropft. Danach werden 10g Tetrachlorcyclopropen in 20 ml glyme bei 0-10°C langsam zugetropft. Die resultierende gelbe Lösung wird noch 1,5 h bei RT gerührt. Der gelbe Niederschlag wird abgesaugt und gewaschen. Das Produkt wird in Wasser gelöst und mit HCl ausgefällt, filtriert, mit Wasser gewaschen und getrocknet sowie aus Tetrachlorkohlenstoff umkristallisiert.
Ausbeute: 60%, Fp.: 121-3°C

2g des Produktes werden portionsweise zu einer gerührten Lösung aus 2g Natriumperiodat in 100ml Wasser gegeben und weiterhin gerührt. Den leuchtend gelben Niederschlag mit Methylenchlorid extrahieren, über Magnesiumsulfat trocknen und das Lösungsmittel abziehen. Den Rückstand in Ether aufnehmen, filtrieren und mit Ether waschen sowie aus n-Butylchlorid umtristallisieren.
Ausbeute: 67% , Fp.: 138-40°C

### Matrixmaterialien

In der vorliegenden Erfindung werden geeignete Dotanden für organische halbleitende Materialien wie Lochtransportmaterialen **HT** beschrieben, die üblicherweise in OLEDs oder organischen Solarzellen verwendet werden. Die halbleitenden Materialien sind vorzugsweise intrinsisch lochleitend. Für erfindungsgemäße Dotanden des Oxokohlenstoff- bzw. des Pseudooxokohlenstoff-Typs kann das Folgende gelten.

Das Matrixmaterial kann teilweise (> 10 oder > 25 Gew.-%) oder im Wesentlichen (> 50 Gew.-% oder > 75 Gew.-%)) oder vollständig bestehen aus einem Metallphtalocyanin-Komplex, einem Porphyrin-Komplex, insbesondere Metallporphyrinkomplex, einer Oligothiophen-, Oligophenyl-, Oligophenylenvinylen- oder Oligofluoren-Verbindung, wobei das Oligomere vorzugsweise 2-500 oder mehr, vorzugsweise 2-100 oder 2-50 oder 2-10 monomere Einheiten umfasst. Gegebenenfalls kann das Oligomer auch > 4, > 6 oder > 10 oder mehr monomere Einheiten umfassen, insbesondere auch für die oben angegebenen Bereiche, also beispielsweise 4 oder 6-10 monomere Einheiten, 6 oder 10-100 monomere Einheiten oder 10-500 monomere Einheiten. Die Monomere bzw. Oligomere können substituiert oder unsubstituiert sein, wobei auch Block- oder Mischpolymerisate aus den genannten Oligomeren vorliegen können, sowie eine Verbindung mit einer Triarylamin-Einheit oder eine Spiro-Bifluoren-Verbindung. Die genannten Matrixmaterialien können auch in Kombination miteinander vorliegen, gegebenenfalls auch in Kombination mit anderen Matrixmaterialien. Die Matrixmaterialien können elektronenschicbende Substituenten wie Alkyl- oder Alkoxy-Reste aufweisen, die eine verminderte Ionisierungsenergie aufweisen oder die Ionisierungsenergie des Matrixmaterials vermindern.

Die als Matrixmaterial eingesetzten Metallphtalocyaninkomplexe oder Porphyrinkomplexe können ein Hauptgruppenmetallatom oder Nebengruppenmetallatom aufweisen. Das Metallatom Me kann jeweils 4-, 5- oder 6-fach koordiniert sein, beispielsweise in Form von Oxo- (Me=O), Dioxo- (O=Me=O), Imin-, Diimin-, Hydroxo-, Dihydroxo-, Amino- oder Diaminokomplexen, ohne hierauf beschränkt zu sein. Der Phtalocyaninkomplex oder Porphyrinkomplex kann jeweils teilweise hydriert sein, wobei jedoch vorzugsweise das mesomere Ringsystem nicht gestört wird. Die Phthalocyaninkomplexe können als Zentralatom beispielsweise Magnesium, Zink, Eisen, Nickel, Kobalt, Magnesium, Kupfer oder Vanadyl (= VO) enthalten. Die gleichen oder andere Metallatome bzw. Oxometallatome können im Falle von Porphyrinkomplexen vorliegen.

Insbesondere können solche dotierbaren Lochtransportmaterialen **HT** arylierte Benzidine, beispielsweise N,N'-perarylierte Benzidine oder andere Diamine wie des Typs **TPD** (wobei eine, mehrere oder sämtliche der Arylgruppen aromatische Heteroatome aufweisen können), geeignete arylierte Starburst-Verbindungen wie N,N',N"-perarylierte Starburstverbindungen, wie die Verbindung **TDATA** (wobei eine, mehrere oder sämtliche der Arylgruppen aromatische Heteroatome aufweisen können), sein. Die Arylreste können insbesondere für jede der oben genannten Verbindungen Phenyl, Naphthyl, Pyridin, Chinolin, Isochinolin, Peridazin, Pyrimidin, Pyrazin, Pyrazol, Imidazol, Oxazol, Furan, Pyrrol, Indol oder dergleichen umfassen. Die Phenylgruppen der jeweiligen Verbindungen können durch Thiophengruppen teilweise oder vollständig ersetzt sein.

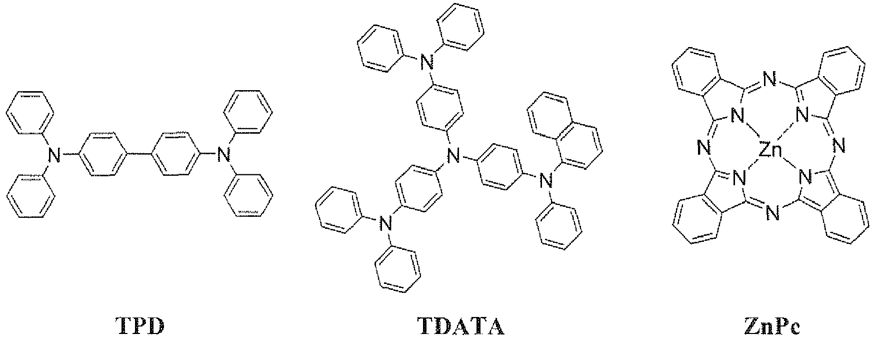

Vorzugsweise besteht das verwendete Matrixmaterial vollständig aus einem Metallphtalocyanin-Komplex, einem Porphyrin-Komplex, einer Verbindung mit einer Triarylamin-Einheit oder einer Spiro-Bifluoren-Verbindung.

Es versteht sich, dass auch geeignete andere organische Matrixmaterialien, insbesondere lochleitende Materialien verwendet werden können, die halbleitende Eigenschaften aufweisen.

### Dotierung

Die Dotierung kann insbesondere derart erfolgen, dass das molare Verhältnis von Dotand zu Matrixmolekül oder im Falle von oligomeren Matrixmaterialien das Verhältnis von Dotand zu Matrixmonomerenanzahl 1:100000, vorzugsweise 1:1 bis 1:10000, besonders bevorzugt 1:5 bis 1:1000 beispielsweise 1:10 bis 1:100, beispielsweise ca. 1:50 bis 1:100 oder auch 1:25 bis 1:50 beträgt.

### Verdampfung der Dotanden

Die Dotierung des jeweiligen Matrixmaterials (hier vorzugsweise angegeben als löcherleitendes Matrixmaterial HT) mit den erfindungsgemäß zu verwendenden Dotanden kann durch eines oder eine Kombination der folgenden Verfahren hergestellt wird:
a) Mischverdampfung im Vakuum mit einer Quelle für HT und einer für den Dotanden.
b) Sequentielles Deponieren von HT und Dotand mit anschliessender Eindiffusion des Dotanden durch thermische Behandlung
c) Dotierung einer HT-Schicht durch eine Lösung von Dotanden mit anschliessendem Verdampfen des Lösungsmittels durch thermische Behandlung
d) Oberflächendotierung einer HT-Schicht durch eine oberflächlich aufgebrachte Schicht von Dotanden

Die Dotierung kann derart erfolgen, dass der Dotand aus einer Precursor-Verbindung heraus verdampft wird, die beim Erhitzen und/oder Bestrahlung den Dotanden freisetzt. Die Bestrahlung kann mittels elektromagnetischer Strahlung, insbesondere sichtbarem Licht, UV-Licht oder IR-Licht erfolgen, beispielsweise jeweils Laserlicht, oder auch durch andere Strahlungsarten. Durch die Bestrahlung kann im wesentlichen die zur Verdampfung notwendige Wärme bereitgestellt werden, es kann auch gezielt in bestimmte Banden der zu verdampfenden Verbindungen bzw. Precursor oder Verbindungskomplexe wie Charge-Transfer-Komplexe eingestrahlt werden, um beispielsweise durch Überführung in angeregte Zustände die Verdampfung der Verbindungen durch Dissoziation der Komplexe zu erleichtern. Es versteht sich, dass die nachfolgend beschriebenen Verdampfungsbedingungen sich auf solche ohne Bestrahlung richten und für Vergleichszwecke einheitliche Verdampfungsbedingungen heranzuziehen sind.

Als Precursorverbindungen können beispielsweise zum Einsatz kommen:
a) Gemische oder stöchiometrische oder mischkristalline Verbindungen aus dem Dotand und einer inerten, nicht-flüchtigen Substanz, z.B. einem Polymer, Molsieb, Aluminiumoxid, Kieselgel, Oligomeren oder einer anderen organischen oder anorganischen Substanz mit hoher Verdampfungstemperatur, wobei der Dotand vorwiegend durch van-der-Waals Kräfte und/oder Wasserstoffbrückenbindung an dieser Substanz gebunden ist.
b) Gemisch oder stöchiometrische oder mischkristalline Verbindung aus dem Dotanden und einer mehr oder weniger Elektronendonor-artigen, nicht flüchtigen Verbindung V, wobei ein mehr oder weniger vollständiger Ladungstransfer zwischen dem Dotanden und der Verbindung V auftritt, wie in Charge-Transfer-Komplexen mit mehr oder weniger elektronenreichen Polyaromaten oder Heteroaromaten oder einer anderen organischen oder anorganischen Substanz mit hoher Verdampfungstemperatur.
c) Gemisch oder stöchiometrische oder mischkristalline Verbindung aus dem Dotanden und einer Substanz, welche zusammen mit dem Dotanden verdampft und eine gleiche oder höhere Ionisierungsenergie aufweist wie die zu dotierende Substanz HT, so dass die Substanz in dem organischen Matrixmaterial keine Haftstelle für Löcher bildet. Hierbei kann die Substanz erfindungsgemäß auch mit dem Matrixmaterial identisch sein, beispielsweise ein Metallphtalocyanin oder Benzidin-Derivat darstellen. Weitere geeignete flüchtige Co-Substanzen, wie Hydrochinone, 1,4-Phenylendiamine oder 1-Amino-4-hydroxybenze oder sonstige Verbindungen bilden Chinhydrone oder andere Charge-Transfer-Komplexe.

### Elektronisches Bauelement

Unter Verwendung der erfindungsgemäßen organischen Verbindungen zur Herstellung dotierter organischer halbleitender Materialien, die insbesondere in Form von Schichten oder elektrischen Leitungspfaden angeordnet sein können, können eine Vielzahl elektronischer Bauelemente oder diese enthaltende Einrichtungen hergestellt werden. Insbesondere können die erfindungsgemäßen Dotanden zur Herstellung von organischen lichtemitierenden Dioden (OLED), organischen Solarzellen, organischen Dioden, insbesondere solchen mit hohem Gleichrichtungsverhältnis wie 10³-10⁷, vorzugsweise 10⁴-10⁷ oder 10⁵-10⁷ oder organischen Feldeffekttransistoren verwendet werden. Durch die erfindungsgemäßen Dotanden kann die Leitfähigkeit der dotierten Schichten und/oder die Verbesserung der Ladungsträgerinjektion von Kontakten in die dotierte Schicht verbessert werden. Insbesondere bei OLEDs kann das Bauelement eine pin-Struktur oder eine inverse Struktur aufweisen, ohne hierauf beschränkt zu sein. Die Verwendung der erfindungsgemäßen Dotanden ist jedoch auf die oben genannten vorteilhaften Ausführungsbeispiele nicht beschränkt.

### Beispiele (nicht erfindungsgemäß)

Die vorstehend angegebenen Verbindungen aus der vorstehend beschriebenen Stoffklasse der Oxokohlen- bzw. der Pseudooxokohlenstoffverbindungen werden nun in folgender Weise als Dotanden für verschiedene Lochleiter verwendet, die ihrerseits zum Aufbau bestimmter mikroelektronischer oder optoelektronischer Bauelemente, wie z.B. einer OLED, benutzt werden. Hierbei können die Dotanden gleichzeitig nebeneinander mit dem Lochtransportmaterialien der Matrix im Hochvakuum (ca. 2x 10⁻⁴Pa) bei erhöhten Temperaturen verdampft werden. Eine typische Substratbedampfungsrate für das Matrixmaterial ist 0,2 nm/s (Dichte ca. 1,5 g/cm³). Die Bedampfungsraten für die Dotanden können variieren zwischen 0.001 und 0.5 nm/s bei gleicher angenommener Dichte, jeweils entsprechend dem gewünschten Dotierungsverhältnis.

In den folgenden Beispielen wurden die Strommessungen über einen 1 mm langen und ca. 0,5 mm breiten Strompfad aus dem dotierten HT-Material bei IV durchgeführt. Unter diesen Bedingungen leitet ZnPc praktisch keinen elektrischen Strom.

Beispiele:

### Beispiel 1

Dotierung von ZnPc mit Dicyanomethylenbis(4-oxo-[3,5-di-*t*-butyl]-2,5-cyclohexadienyliden)cyclopropan
Leitfähigkeit: 1,5 x 10⁻⁵S/cm

### Beispiel 2

Dotierung von Spiro-TTP mit Dicyanomethylenbis(4-oxo-[3,5-di-*t*-butyl]-2,5-cyclohexadienyliden)cyclopropan
Leitfähigkeit: 3, 6 x 10⁻⁷S/cm

### Beispiel 3

Dotierung von ZnPC mit 1,3-Bis(dicyanomethylen)indan-2-yliden-bis(4-oxo-[3,5-di-*t*-butyl]-2,5-cyclohexadienyliden)cyclopropan Leitfähigkeit: 5,8 x 10⁻⁶S/cm

### Beispiel 4

Dotierung von Spiro-TTP mit 1,3-Bis(dicyanomethylen)indan-2-yliden-bis(4-oxo-[3,5-di-*t-*butyl]-2,5-cyclohexadienyliden)cyclopropan Leitfähigkeit: 5 x 10⁻⁷S/cm

## Patentansprüche

1. Verwendung einer organischen mesomeren Verbindung als organischer Dotand zur Dotierung eines organischen Lochtransportmaterials oder als Ladungsinjektionsschicht **dadurch gekennzeichnet, dass** die mesomere Verbindung eine Oxokohlenstoff- , Pseudooxokohlenstoff- oder Radialenverbindung der folgenden Formel ist: 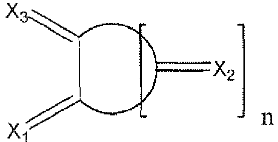 wobei n = 1 ist; jedes X₁, X₂ und X₃, jeweils unabhängig ausgewählt wird aus der Gruppe bestehend aus 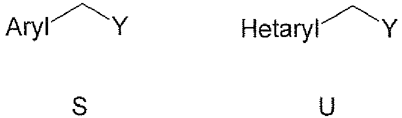 wobei Y = CN, NO₂, COR₁ oder perhalogeniertes Alkyl ist; Aryl ein substituierter oder unsubstituierter aromatischer Kohlenwasserstoff oder Biaryl ist, ggf. polycyclisch; Hetaryl eine substituierte oder unsubstituierte aromatische heterocyclische Verbindung oder Biheteroaryl ist, vorzugsweise elektrodenarm, ggf. mehrkernig oder teilweise o-der vollständig hydriert bzw. fluoriert; und R₁ ausgewählt wird aus Wasserstoff, Halogen, CN, NO₂, Alkyl, Alkoxy, Aryl und Heteroaryl.

2. Verwendung nach Anspruch 1, **dadurch gekennzeichnet, dass** Y Perfluoralkyl, wie CF₃, ist.

3. Verwendung nach Anspruch 1 oder 2, **dadurch gekennzeichnet, dass** Aryl teilweise oder vollständig hydriert bzw. fluoriert ist.

4. Verwendung nach einem der vorangehenden Ansprüche, **dadurch gekennzeichnet, dass** Hetaryl ausgewählt wird aus Pyridyl, Pyrimidyl, Triazin und Oxadiazol.

5. Verwendung nach einem der vorangehenden Ansprüche, **dadurch gekennzeichnet, dass** R₁ ausgewählt wird aus perhalogenierten und/oder teilweise halogenierten Alkylgruppen, insbesondere perfluorierten Alkylgruppen.

6. Oxokohlenstoff-, Pseudooxokohlenstoff- oder Radialenverbindung der folgenden Formel 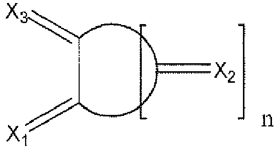 wobei n = 1 ist;
wobei jedes X₁-X₃ jeweils unabhängig ausgewählt wird aus 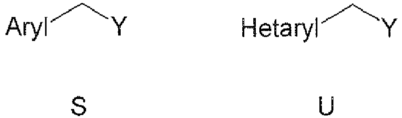 Y ausgewählt wird aus CN, COR₁, perhalogenierten Alkylgruppen, insbesondere Perfluoralkyl; Aryl ausgewählt wird aus substituiertem oder unsubstituiertem aromatischen Kohlenwasserstoff, auch polycyclisch, und Biaryl, teilweise oder vollständig hydriert bzw. fluoriert; Hetaryl ausgewählt wird aus substituierter oder unsubstituierter aromatischer heterocyclischer Verbindung, vorzugsweise elektronenarmer Verbindung, auch mehrkernig, und auch teilweise oder vollständig hydrierten bzw. fluorierten Heteroaromaten; R₁ ausgewählt wird aus Wasserstoff, Halogen, CN, NO₂, perhalogenierten und/oder teilweise halogenierten Alkylgruppen, substituierten und/oder unsubstituierten Aryl- und Heteroarylgruppen;
wobei die folgende Verbindung ausgeschlossen ist:
a) n = 2; X₁ = X₂ = X₃ = Unterstruktur S, Ar = Phenyl, Y = CF₃.

7. Oxokohlenstoff-, Pseudooxokohlenstoff- und Radialenverbindungen nach Anspruch 6, **dadurch gekennzeichnet, dass** Hetaryl ausgewählt wird aus Pyridyl, Pyrimidyl, Triazin und Oxadiazol.

8. Verwendung der Oxokohlenstoff-, Pseudooxokohlenstoff- und Radialenverbindungen nach Anspruch 6 oder 7 oder ihrer Radikalanionensalze oder Dianionensalze als organische Ferromagneten.

9. Verwendung der Oxokohlenstoff-, Pseudooxokohlenstoff- und Radialenverbindungen nach einem der Ansprüche 6 oder 7 oder ihrer Radikalanionensalze als organische Leiter.

10. Organisches halbleitendes Material enthaltend zumindest eine organische Matrixvebindung und einen Dotanden, **dadurch gekennzeichnet, dass** der Dotand eine oder mehrere Oxokohlenstoff-, Pseudooxokohlenstoff- oder Radialenverbindungen gemäß einem der Ansprüche 1 bis 5 ist.

11. Organisches halbleitendes Material nach Anspruch 10, **dadurch gekennzeichnet, dass** das molare Dotierungsverhältnis von Dotand zu Matrixmolekül bzw. das Dotierungs-Verhältnis von Dotand zu monomeren Einheiten eines polymeren Matrixmoleküls 1:5 bis 1:1000 beträgt.

12. Elektronisches Bauelement mit einem elektronisch funktionell wirksamen Bereich, **dadurch gekennzeichnet, dass** der elektronisch wirksame Bereich unter Verwendung zumindest einer oder mehrerer Oxokohlenstoff-, Pseudooxokohlenstoff- und Radialenverbindungen nach einem der Ansprüche 1 bis 5 hergestellt ist.

13. Elektronisches Bauelement nach Anspruch 12, **dadurch gekennzeichnet, dass** der elektronisch wirksame Bereich ein organisches halbleitendes Matrixmaterial aufweist, welches mit zumindest einem Dotanden zur Veränderung der elektronischen Eigenschaften des halbleitenden Matrixmaterials unter Verwendung zumindest einer oder mehrerer der Oxokohlenstoff-, Pseudooxokohlenstoff- und Radialenverbindungen nach einem der Ansprüche 1 bis 5 dotiert ist.

14. Elektronisches Bauelement nach Anspruch 12 oder 13 in Form einer organischen lichtemittierenden Diode, einer photovoltaischen Zelle, einer organischen Solarzelle, einer organischen Diode oder eines organischen Feldeffekttransistors.

## Claims

1. Use of an organic mesomeric compound as organic doping agent for the doping of an organic hole transporting material or as charge injection layer, **characterized in that** the mesomeric compound is an oxocarbon-, pseudooxocarbon- or radialene compound with the following formula: 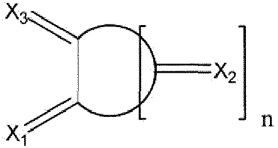 wherein n = 1; each X₁, X₂ and X₃ is independently selected from the group consisting of 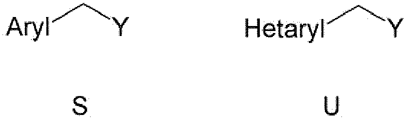 wherein Y = CN, NO₂, COR₁ or is perhalogenated alkyl; aryl is a substituted or unsubstituted aromatic hydrocarbon or biaryl, optionally polycyclic; hetaryl is a substituted or unsubstituted aromatic heterocyclic compound or biheteroaryl, preferably electron-poor, optionally polynuclear or partially or completely hydrogenated or fluorinated; and R₁ is selected from hydrogen, halogen, CN, NO₂, alkyl, alkoxy, aryl and heteroaryl.

2. Use according to claim 1, **characterized in that** Y is perfluoroalkyl, such as CF₃.

3. Use according to claim 1 or 2, **characterized in that** aryl is partially or completely hydrogenated or fluorinated.

4. Use according to any of the preceding claims, **characterized in that** the hetaryl is selected from pyridyl, pyrimidyl, triazine and oxadiazole.

5. Use according to any of the preceding claims, **characterized in that** R₁ is selected from perhalogenated and/or partially halogenated alkyl groups, especially perfluorinated alkylgroups.

6. Oxocarbon-, pseudooxocabon- or radialene compound with the following formula 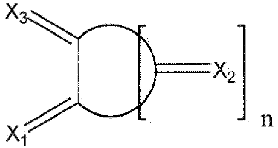 wherein n = 1;
wherein each X₁ to X₃ is independently selected from 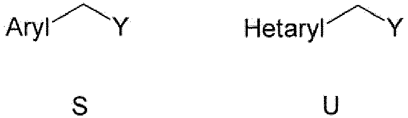 Y is selected from CN, COR₁, perhalogenated alkyl groups, especially perfluoroalkyl; aryl is selected from substituted or unsubstituted aromatic hydrocarbon, especially polycyclic, and biaryl, partially and completely hydrogenated and fluorinated; hetaryl is selected from substituted or unsubstituted aromatic heterocyclic compound, preferably electron-poor compound, also polynuclear and also partially or completely hydrogenated or fluorinated heteroaromatics: R₁ is selected from hydrogen, halogen, CN, NO₂, perhalogenated and/or partially halogenated alkyl groups, unsubstituted and/or substituted aryl- and heteroaryl groups;
wherein the following compound is excluded:
a) n = 2; X₁ = X₂ = X₃ = substructure S, Ar = phenyl, Y = CF₃.

7. Oxocarbon-, pseudooxocarbon- and radialene compound according to claim 6, **characterized in that** hetaryl is selected from pyridyl, pyrimidyl, triazine and oxadiazole.

8. Use of the oxocarbon-, pseudooxocarbon-, and radialene compounds according to claim 6 or 7 or their radical anionic salts or dianioic salts as organic ferromagnets.

9. Use of oxocarbon-, pseudooxocarbon- and radialene compounds according to claim 6 or 7 or radical anionic salts thereof as organic conductors.

10. Organic semiconductive material containing at least one organic matrix compound and one doping agent, **characterized in that** the doping agent is one or more oxocarbon-, pseudooxocarbon- or radialene compound according to one of claims 1 to 5.

11. Organic semiconductive material according to claim 10, **characterized in that** the molar doping ratio of doping agent to matrix molecule and/or the doping ratio of doping agent to monomeric unit of a polymeric matrix molecule is between 1:5 and 1:1000.

12. Electronic component with an electronically functionally active area, **characterized in that** the electronically functionally active area is produced using at least one or more oxocarbon-,pseudooxocarbon- or radialene compound according to one of the claims 1 to 5.

13. Electronic component according to claim 12, **characterized in that** the electronically functionally active area comprises an organic semiconductive matrix material that is doped with at least one doping agent for changing the electronic properties of the semiconductive matrix material using at least one or more of the oxocarbon-, pseudooxocarbon- or radialene compounds according to one of claims 1 to 5.

14. Electronic component according to claim 12 or 13 in the form of an organic light emitting diode, a photovoltaic cell, an organic solar cell, an organic diode or an organic field effect transistor.

## Revendications

1. Utilisation d'un composé mésomère organique en tant que dopant organique destiné à doper un matériau organique de transport de trous ou en tant que couche d'injection de charges, **caractérisée en ce que** ledit composé mésomère est un composé oxycarboné, pseudo-oxycarboné ou de type radialène répondant à la formule suivante : 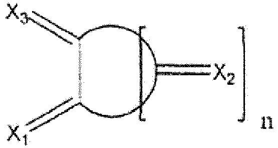 n étant égal à 1 ; chaque X₁, X₂ et X₃ étant choisi de façon indépendante dans le groupe constitué de 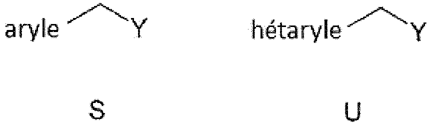 Y étant égal à CN, NO₂, COR₁ ou alkyle perhalogéné ; aryle étant un hydrocarbure aromatique substitué ou non-substitué ou un biaryle, éventuellement de nature polycyclique ; hétaryle étant un composé hétérocyclique aromatique substitué ou non-substitué ou un bihétéroaryle, de préférence pauvre en électrons, éventuellement à plusieurs noyaux ou de nature partiellement ou complètement hydrogénée ou fluorée ; et R₁ étant choisi parmi hydrogène, halogène, CN, NO₂, alkyle, alkoxy, aryle et hétéroaryle.

2. Utilisation selon la revendication 1, **caractérisée en ce que** Y représente un perfluoroalkyle tel que CF₃.

3. Utilisation selon les revendications 1 ou 2, **caractérisée en ce que** ledit aryle est de nature partiellement ou complètement hydrogénée ou fluorée.

4. Utilisation selon l'une des revendications précédentes, **caractérisée en ce que** ledit hétaryle est choisi parmi le pyridyle, le pyrimidyle, la triazine et l'oxadiazole.

5. Utilisation selon l'une des revendications précédentes, **caractérisée en ce que** R₁ est choisi parmi les groupes alkyle perhalogénés et/ou partiellement halogénés, notamment les groupes alkyle perfluorés.

6. Composé oxycarboné, pseudo-oxocarboné ou de type radialène répondant à la formule suivante 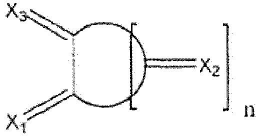 n étant égal à 1 ;
chaque X₁-X₃ étant choisi de façon indépendante parmi 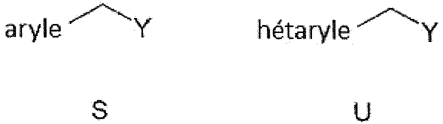 Y étant choisi parmi CN, COR₁, des groupes alkyle perhalogénés, notamment perfluoroalkyle; aryle étant choisi parmi un hydrocarbure aromatique substitué ou non-substitués, éventuellement de nature polycyclique, et un biaryle, de nature partiellement ou complètement hydrogénée ou fluorée ; hétaryle étant choisi parmi un composé hétérocyclique aromatique substitué ou non-substitué, s'agissant préférentiellement d'un composé pauvre en électrons, éventuellement à plusieurs noyaux, et également parmi les hétéroaromates de nature partiellement ou complètement hydrogénée ou fluorée ; R₁ étant choisi parmi hydrogène, halogène, CN, NO₂, des groupes alkyle perhalogénés et/ou partiellement halogéné, des groupes aryle et hétéroaryle substitués et/ou non-substitués ;
le composé suivant étant exclu :
a) n = 2 ; X₁ = X₂ = X₃ = sous-structure S, Ar = phényle, Y = CF₃.

7. Composés oxycarbonés, pseudo-oxocarbonés ou de type radialène selon la revendication 6, **caractérisés en ce que** ledit hétaryle est choisi parmi le pyridyle, le pyrimidyle, la triazine et l'oxadiazole.

8. Utilisation des composés oxycarbonés, pseudo-oxocarbonés ou de type radialène selon les revendications 6 ou 7, ou de leurs sels d'anions radicalaires ou sels de dianions, en tant que corps ferromagnétiques organiques.

9. Utilisation des composés oxycarbonés, pseudo-oxocarbonés ou de type radialène selon les revendications 6 ou 7, ou de leurs sels d'anions radicalaires, en tant que conducteurs organiques.

10. Matériau semi-conducteur organique, contenant au moins un composé de matrice organique et un dopant, **caractérisé en ce que** ledit dopant correspond à un ou plusieurs composés oxycarbonés, pseudo-oxocarbonés ou de type radialène selon l'une des revendications 1 à 5.

11. Matériau semi-conducteur organique selon la revendication 10, **caractérisé en ce que** le rapport molaire de dopage entre le dopant et la molécule de matrice, ou le rapport de dopage entre le dopant et les unités monomères d'une molécule de matrice polymère, est compris entre 1 : 5 et 1 : 1000.

12. Composant électronique comportant une zone ayant un effet électroniquement fonctionnel, **caractérisé en ce que** la zone ayant un effet électronique est réalisée en utilisant au moins un ou plusieurs composés oxycarbonés, pseudo-oxocarbonés ou de type radialène selon l'une des revendications 1 à 5.

13. Composant électronique selon la revendication 12, **caractérisé en ce que** ladite zone ayant un effet électronique comporte un matériau de matrice semi-conducteur organique lequel est dopé avec au moins un dopant pour ainsi modifier les propriétés électroniques dudit matériau de matrice semi-conducteur en utilisant au moins un ou plusieurs des composés oxycarbonés, pseudo-oxocarbonés ou de type radialène selon l'une des revendications 1 à 5.

14. Composant électronique selon les revendications 12 ou 13 sous forme d'une diode électroluminescente organique, d'une cellule photovoltaïque, d'une cellule solaire organique, d'une diode organique ou d'un transistor à effet de champ organique.
